# EUROPEAN PATENT SPECIFICATION

(11) **EP 1 596 712 B1**
(45) Date of publication and mention of the grant of the patent: **07.11.2007**
(21) Application number: 04710682.8
(22) Date of filing: 12.02.2004
(51) Int. Cl.: A61B 5/00, G01N 21/64

(54) **CHEMICAL SENSOR**
CHEMISCHER SENSOR
DETECTEUR CHIMIQUE

(30) Priority: 13.02.2003 US 365798
(43) Date of publication of application: 23.11.2005
(73) Proprietor: MEDTRONIC, INC., Minneapolis, MN 55432 (US)
(72) Inventor: YANG, Zhongping, Woodbury, MN 55129 (US); MARTIN, Roy, C., Maple Grove, MN 55311 (US); CARNEY, James, K., Eden Prairie, MN 55347 (US)
(74) Representative: Hughes, Andrea Michelle
(86) International application number: PCT/US2004/004210
(87) International publication number: WO 2004/071291

(56) References cited:
- EP-A- 0 834 734
- WO-A-99/49302
- US-A- 5 833 603
- US-A1- 2001 034 479
- US-B1- 6 304 766

## Description

The present invention generally relates to sensors for measuring one or more parameters of a sample fluid, and more particularly relates to implantable sensors for in situ measurement of parameters of body fluids.

It is known in the art to use electro-optical sensing devices for detecting the presence or concentration of an analyte (i.e., a parameter to be measured) in a liquid or gaseous medium. For example, U. S. Patent Numbers 6,454,710, 6,330,464, 6,304,766 6,119,028, 6,081,736, 5,910,661, 5,894,351, 5,728,422 and WO-A-99/49302 describe various aspects of this technology including self-contained sensing devices stated to be suitable for use in humans for in-situ detection of various analytes.

Compact chemical sensors able to detect analytes of interest in connection with human and animal health are of great practical utility, especially arrangements that are implantable. The most common optical chemical sensor configuration uses an LED to provide stimulating light that is optically coupled to an indicator layer in or on which are placed molecules whose fluorescence or absorption is affected by the presence of the analyte, that is, the parameter desired to be measured. A photodetector is likewise optically coupled to the indicator layer for detecting the amount of fluorescence, fluorescence quenching or absorption created by the interaction of the indicator molecules in the presence of the stimulating light and the analyte. In this way, the concentration of sugars, oxygen and other parameters or analytes of interest can be measured in situ. Various configurations and arrangements are used for coupling the stimulating light to the indicator molecules and the optical response thereof back to the photodetectors, as for example, optical fibers or wave guides, or optically transparent encapsulating material. Document WO-A-99/49302 shows a sensor as defined in the preamble of claim 1.

However the prior art devices and methods suffer from a number of limitations, as for example, high cost, fragile construction, difficult manufacturing, inefficient optical coupling and limited sensitivity under some circumstances.

The invention provides a chemical sensor for detecting or measuring a parameter of interest in a sample fluid, comprising:
a housing chemically resistant to the sample fluid;
a sensor element substantially within the housing, the sensor element having a sensing layer and an optically transparent body with a first surface exposed from the housing for receiving a sample responsive to the parameter of interest in the sample fluid and for receiving a first signal from the sensing layer in response to optical stimulation thereof;
wherein the sensor element comprises a light emitter on a second surface of the body, optically coupled to the first surface for providing said optical stimulation;
wherein the sensor element comprises a first detector on a third surface of the body, optically coupled to the first surface for detecting said first signal; and characterized in that of the first, second and third surfaces of the body, two surfaces are opposed and a remaining surface couples the opposed surfaces.

The chemical sensor of the invention is an improved sensor, especially an improved implantable sensor, that is rugged, highly integrated, easily manufactured and functional for detecting the presence and/or amount of various analytes or parameters in situ in human and animal bodies and that generate little or no adverse reactions within the body.

The present invention will hereinafter be described in conjunction with the following drawing figures, wherein like numerals denote like elements, and
FIG. 1 is a simplified schematic cross-sectional view of an implantable chemical sensor according to the present invention;
FIGS. 2-4 are a simplified schematic side cross-sectional views of the opto-electronic portion of the sensor of FIG. 1, showing further details and according to several embodiments;
FIGS. 5A-B through 8A-B are simplified side views (A) and bottom views (B) of the opto-electronic portion of FIGS. 2-4, showing further details and according to still further embodiments; and
FIG. 9 is a simplified schematic cross-sectional view similar to FIG. 1 of an implantable chemical sensor according to a still further embodiment of the present invention.

The following detailed description of the invention is merely exemplary in nature and is not intended to limit the invention or the application and uses of the invention. Persons of skill in the art will understand the general principles of operation of optically stimulated chemical sensors and the sensing layer materials commonly used. Any particular choice of sensing materials and sensing layer structures depends on the particular analytes of interest. This invention is particularly concerned with the efficient coupling of light to and from the sensing layer and providing a particularly rugged, efficient, easily manufactured and compact chemical sensor structure.

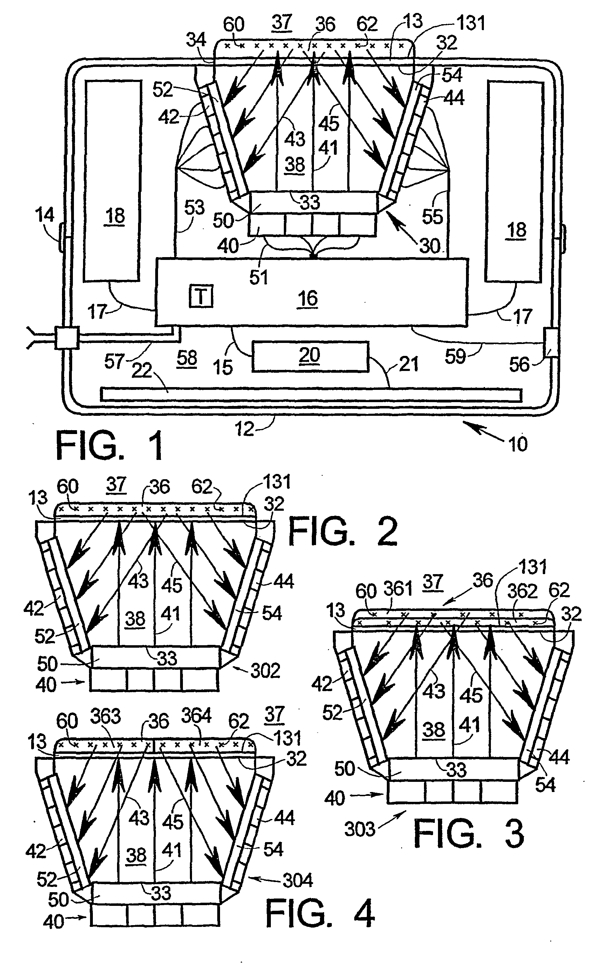

FIG. 1 is a simplified schematic cross-sectional view of implantable chemical sensor 10 according to the present invention. Chemical sensor 10 comprises hermetic enclosure or housing 12 that is sealable along joint 14. Conveniently located within housing 12 are optical module 30, electronic control module 16, energy source 18 (e.g., one or more batteries), optional transceiver 20, optional antenna 22 and optional pressure sensor 56 having a sensing diaphragm communicating with ambient 37 through an external wall of housing 12. Optical module 30 has therein light emitter(s) 40 coupled to control module 16 via leads 51, light detectors 42, 44 coupled to control module 16 via leads 53, 55, respectively, and other components as will be presently described. Control module 16 is coupled to energy source 18 via lead(s) 17 and to transceiver 20 via lead(s) 15. Transceiver 20 is coupled to antenna 22 via lead(s) 21. While transceiver 20 and antenna 22 are conveniently provided for communicating to and from sensor 10 they are not essential and externally coupled lead(s) 57 may also be used to provide bi-directional communications with sensor 10. Further, while it is desirable that instructions or other commands be able to be sent to sensor 10 while in situ using lead(s) 57 or antenna 22, this is not essential. Sensor 10 can be preprogrammed to merely report measured data after implantation without further instructions from outside.

Enclosure or housing 12 protects the internal components from the sample fluid. Housing 12 is conveniently opaque although this is not essential. Housing or enclosure 12 is preferably made of a bio-inert material suitable for long-term placement in a human or animal body. Titanium is an example of a suitable material but others materials will also serve. Temperature sensor [T] is also desirably included in control module 16 or elsewhere in housing 12 so as to provide ambient temperature information for adjusting or compensating the various signals processed by control module 16, but this is not essential. Housing 12 may also be conveniently coated with a layer (not shown) of a chemically inert material such as for example, Teflon® or the like.

Housing 12 has optically transparent window 13 therein with outer surface 131. Window 13 is hermetically attached to housing 12 by, for example, laser welding, soldering or gluing or other appropriate sealing and attachment means well known in the art at, depicted herein for example, perimeter joint 34. Sapphire, quartz, glasses and optically transparent plastics are examples of convenient materials for optical window 13. Other optically materials may also be used. In the preferred embodiment, it is necessary that window 13 be hermetically sealed to housing 12 so as to preclude ambient leakage into housing 12 via the attachment joint (e.g., joint 34) and that window 13 be transparent to the light wavelengths of interest. Window 13 is conveniently joined to or a part of body 38 of optical module 30 at interface 32. Window 13 and body 38 of optical module 30 may be integral, that is, made of a single piece of material, in which case interface 32 is a hypothetical plane internal to body 38, or window 13 and body 38 can be joined at real interface 32, as for example, using optically transparent epoxy or solder glasses or by other means well known in the art. Either arrangement is useful and the choice depends upon convenience of manufacture and overall optical properties. What is important is that the maximum amount of light be coupled from body 38 to and from sensing layer 36 on surface 131. As used herein, the terms "optical body" and "monolithic optical body" are intended to include both arrangements, e.g., attached or integral windows. Window 13 with outer surface 131 on which sensor layer 36 is supported is shown herein as having flat outer surface 131 in contact with sensor layer 36, but this is merely for convenience of explanation and not intended to be limiting. Surface 131 of window 13 can have a curved or of any other shape in contact with sensor layer 36 provided that such shape does not interfere with optical coupling between sensor layer 36 and optical module 30.

Optical module 30 has optically transparent body 38 coupled to or integral with surface 131 on which is located sensing layer 36. Body 38 is conveniently formed of the same class of optically transparent materials as discussed in connection with window 13. Sensing layer 36 is exposed to ambient fluid or gases 37 whose composition or properties are desired to be analyzed. Body 38 of optical module 30 couples light energy 41 to sensing layer 36 from light source 40, e.g., one or more LED's. Light energy 41 is generated by light source(s) 40 and conveniently passes through input filter 50 before entering optical body 38. Input filter 50 is useful for selecting from the light emitted by light source(s) 40, just the wavelengths desired to excite sensing layer 36. While optical filter 50 is desirable it is not essential. Light source(s) 40 may be a single larger area LED or other emitter or an array of smaller emitters, such as for example are shown in FIGS. 1-4. Either arrangement is suitable. While LEDs are a convenient light source and are preferred, other light sources can be used, as for example and not intended to be limiting, electro-luminescent or gas discharge or other types of light sources well known in the art. As used herein, the term "LED" is intended to include these and other types of light sources. While filter 50 is shown as being a single unit, this is not essential and not intended to be limiting. For example, filter 50 may be different over different LEDs making up light source 40 or may have multiple pass-bands so that multiple selected wavelengths emitted from the same or different LEDs are selectively coupled to optical body 38. Thus, LEDs 40 and/or filter 50 need not be restricted to a single wavelength.

Body 38 of module 30 also couples light energy 43, 45 from sensing layer 36 to various photo detectors 42, 44. Light energy 43, 45 may originate from fluorescence generated in sensing layer 36 by light energy 41 or be a portion of light energy 41 being reflected or scattered back from layer 36. Filter 52 is located between photodetector(s) 42 and optical body 38 and filter 54 is conveniently located between photodetector(s) 44 and optical body 38. Filters 52, 54 conveniently attenuate extraneous light so that photodetectors 42, 44 detect and measure only specific desired wavelengths of light 43, 45 respectively. While filters 52, 54 are desirable they are not essential. Filters 52, 54 should have pass-bands matched to the wavelengths desired to be detected by photodetectors 42, 44, respectively. Photodetectors 42, 44 can comprise a single large area photocell or an array of smaller photocells; either arrangement is useful. In FIGS. 1-4, the arrangement using multiple photocells 42, 44 is illustrated, but this is not essential. As used herein, the words "photodetector" and "photodetectors" or "photodetector(s)" are intended to include either arrangement.

While filter-photodetector pairs 42, 52 and 44, 54 are ordinarily designed to, each detect a particular wavelength, this is not essential and the filter-photodetector pairs can be made up of several filter-photodetector elements capable of detecting different wavelengths. For example, under circumstances where it is desired to independently monitor the output of sensor layer 36 at multiple wavelengths, filter-photodetector pair 42, 52 may have a first segment that detects a first wavelength and a second segment that independently detects a second wavelength, thus permitting the intensity of the light of the two wavelengths to be compared. Filter-photodetector pair 44, 54 may be similarly arranged to have multiple independent elements. The present invention and the appended claims are intended to include such variations.

Stimulating light source 40 is coupled to control module 16 by leads 51. Photodetector 42 is coupled to control module 16 by leads 53 and photodetector 44 is coupled to control module 16 by leads 55. The space within housing 12 surrounding and separating components 16, 18, 20, 22, 30 and the various interconnecting leads or buses is conveniently filled with an inert plastic filler or potting material 58 so as to provide ruggedness, mechanical shock resistance, improved hermeticity and electrical insulation. An aspect of the present invention is that filler material 58 need not be optically transparent thereby offering a wider range of material choices and the opportunity to select more favorable mechanical, electrical and optical properties than available in prior art devices where the internal filler or potting material is used as an optical wave guide or optical pathway from the light emitters to the sensing layer and back to the optical detectors.

In operation, stimulating light source 40 operating under the control of electronic module 16 generates light 41 that passes through filter 50 and optical body 38 to sensing layer 36. Sensing layer 36 contains various indicator molecules 60 that, for example, fluoresce when illuminated by light 41. Indicator molecules 60 are sensitive to the analyte desired to be detected in ambient fluids or gases 37. In a typical situation, if the desired analyte is present in ambient 37, the fluorescence, e.g., light 43 emitted by indicator molecules 60, increases or decreases proportional to the concentration of the analyte to which indicator molecules are sensitive. Such indicator molecules are well known in the art and their choice depends upon ambient 37 and the particular analyte desired to be detected. Fluorescent light 43 passes through incoming filter 52 and is measured by photodetector 42. Filter 52 is chosen to pass the wavelength of the fluorescence emitted by indicator molecules 60 and attenuate other wavelengths, e.g., the wavelengths of light 41, 45.

In order to increase the measurement accuracy and compensate for difference in temperature and pressure it is desirable to provide a reference signal to which the analyte sensitive signals registered by photodetector 42 may be compared. This is desirably accomplished by including reference indicator molecules 62 in sensing layer 36. Such reference indicator molecules are well known in the art. Reference indicator molecules 62 are chosen, for example, to fluoresce at a different wavelength than indicator molecules 60 and to be insensitive to the analyte. Such molecules are well known in the art. Reference molecules 62, for example, emit fluorescent light 45 that is coupled though optical body 38 and filter 54 to photodetector 44. Filter 54 desirably attenuates other wavelengths, e.g., of light 41, 43 so that photodetector 44 measures substantially only the output of reference indicator molecules 62. The output of photodetector 44 is coupled via leads 55 to electronic control module 16 where the reference signal received by photodetector 44 is used to compensate the analyte sensitive signal from photodetector 42 for changes in conditions.

Control module 16 conveniently uses transmitter or transceiver 20 to send the test results to antenna 22 where they are broadcast to a receiver outside the body. Persons of skill in the art will understand how to choose transmitter or transceiver frequencies to accomplish this. Similarly, control module 16 can receive program instructions from outside the body via antenna 22 and transceiver 20. In this way, the operation of control module 16 can be externally programmed to, for example, conduct tests at different times or with different repetition rates or different light stimulation levels and other variations. Alternatively or in combination, externally coupled wires 57 may be used to input instructions to sensor module 10 and receive data therefrom. Either arrangement is suitable.

FIGS. 2-4 are simplified schematic side cross-sectional views of optical module 30 of sensor 10 of FIG. 1, showing further details and according to several embodiments 302, 303, 304. Like reference numbers identify like elements. FIGS. 2-4 differ only in the composition and arrangement of sensing layer 36. Other aspects of modules 302, 303, 304 are the same is in module 30 of FIG. 1.

In FIG. 2, sensing layer 36 of optical module 302 has analyte indicator molecules 60 and reference indicator molecules 62 dispersed substantially homogeneously in layer 36. In optical module 303 of FIG. 3, a layered arrangement is used for sensing layer 36 wherein, for example, analyte indicator molecules 60 are dispersed in layer 361 and reference indicator molecules 62 are dispersed in layer 362. In module 303 of FIG. 3, layer 361 is shown as superposed on layer 362 but this is merely for convenience of explanation. Those of skill in the art will understand that the order of layers maybe interchanged depending on the analyte and the reference indicator, that is, with layer 362 outermost and layer 361 innermost. Either arrangement suffices depending on the analyte and reference involved. In optical module 304 of FIG. 4, the regions of sensing layer 36 containing analyte indicator molecules 60 and reference indicator molecules 62 are laterally distinct. In optical module 304, analyte indicator molecules 60 are in region 363 and reference indicator molecules 62 are in region 364, but these regions may be interchanged. Other lateral separation arrangements may also be used, as for example, regions 363 and 364 may be interspersed in alternate stripes or alternate concentric circles or otherwise and FIG. 4 is merely intended to be illustrative of lateral separation and not limited to the particular arrangement shown. Similarly, the arrangements illustrated in FIGS. 2-4 may be used simultaneously or in combination in different regions of window 13.

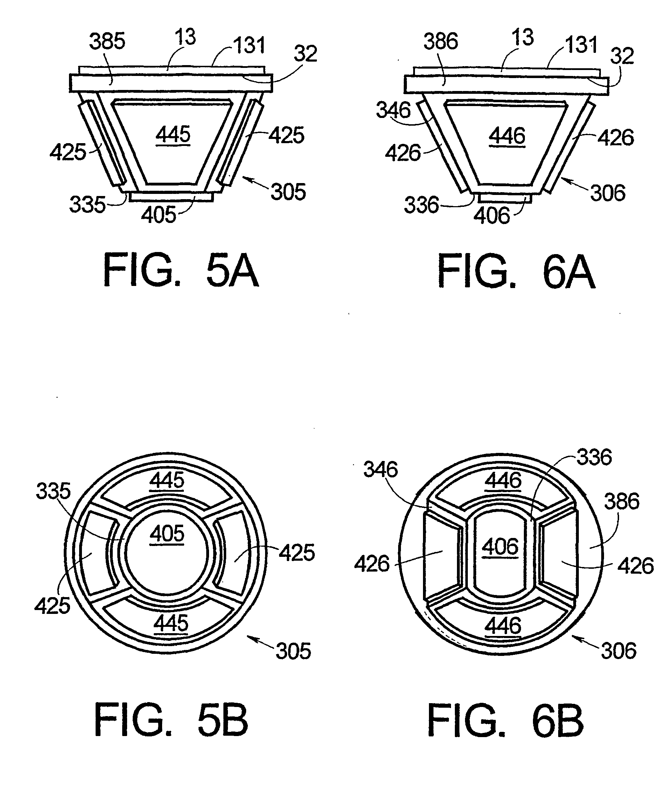

FIGS. 5A-B through 8A-B are simplified side views (A) and bottom views (B) respectively, of optical module 30 of FIGS. 1-4, showing further details and according to still further embodiments 305, 306, 307, 308. Like reference numbers are used for like elements. In FIGS. 5-8, the details of sensing layer 36 are omitted and filter layers 50, 52, 54 are not shown, but this is merely for convenience of explanation and not intended to be limiting. Persons of skill in the art will understand based on the description herein that the variations illustrated in FIGS. 2-4 also apply to optical sensing modules 305-308 of FIGS. 5-8. FIGS. 5-8 illustrate various alternative arrangements 305, 306, 307, 308 of optical sensing module 30 with different location and shapes of photodetector 42 for receiving analyte sensing light signal 43 and photodetector 44 for receiving the reference light signal 45 and photo-emitter 40 for generating stimulating light 41. For convenience of explanation and not intended to be limiting, in FIGS. 5-8, variations of optical sensing modules 30 are identified by reference numerals 305, 306, 307, 308, variations of analyte signal detector 42 are identified by reference numerals 425, 426, 427, 428, variations of reference signal detector 44 are identified by reference numerals 445, 446, 447, 448, variations of stimulating light emitter 40 are identified by reference numerals 405, 406, 407, 408, variations of optical body 38 are identified by reference numerals 385, 386, 387, 388 and variations of lower surface 33 of optical body 38 are identified by reference numerals 335, 336, 337, 338. For simplicity, sensing layer 36 and indicator molecules 60, 62 are omitted from FIGS. 5-8 but those of skill in the art will understand that such are present on window 13 when optical module 30 is present in housing 12. The explanations provided earlier about elements 30, 33, 38, 42, 44 also apply to the variations identified here.

FIG. 5A is a side view and FIG. 5B is a bottom view of optical sensing module 305 showing opposed pairs of photodetectors 425 and opposed pairs of photodetectors 445. Stimulating light emitter 405 is located on bottom surface 335 of electro-optic module 305 opposite outer surface 131 of window 13 on which sensing layer 36 (not shown) would be located. Optical body 385 has a generally truncated cone shape with the larger diameter upper portion mating with housing 12 and surface 131 for supporting sensing layer 36. Having the photodetectors arranged in opposing pairs increases the optical coupling through optical body 385 and provides a strong signal. FIG. 6A is a side view and FIG. 6B is a bottom view of optical sensing module 306 showing opposed pairs of photodetectors 426 and opposed pairs of photodetectors 446. Stimulating light emitter 406 is located on bottom surface 336 of electro-optic module 306 opposite outer surface 131 on which sensing layer 36 (not shown) would be located. Optical body 386 has a generally truncated cone shape with the larger diameter upper portion mating with housing 12 and surface 131 for supporting sensing layer 36. The lower, cone-shaped portion has two curved sides on which detectors 426 are located and two flat sides 346 on which detectors 426 are located. Having the photodetectors arranged in opposing pairs increases the optical coupling through optical body 386 and provides a strong signal.

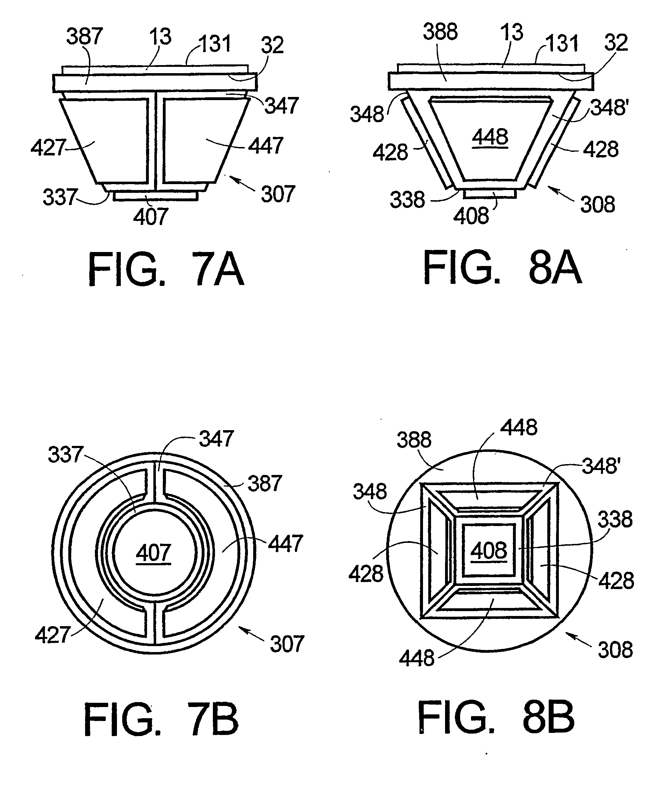

FIG. 7A is a side view and FIG. 7B is a bottom view of optical sensing module 307 showing photodetectors 427 and photodetectors 447 in an opposed arrangement. Stimulating light emitter 407 is located on bottom surface 337 of electro-optic module 307 opposite outer surface 131 of window 13 on which sensing layer 36 (not shown) would be located. Optical body 387 has a generally truncated cone shape with the larger diameter upper portion mating with housing 12 and surface 131 for supporting sensing layer 36. Having the photodetectors arranged so as to lap substantially around side 347 of the cone shape increases the optical coupling through optical body 387 and provides a strong signal.

FIG. 8A is a side view and FIG. 8B is a bottom view of optical sensing module 308 showing opposed pairs of photodetectors 428 and opposed pairs of photodetectors 448. Stimulating light emitter 408 is located on bottom surface 338 of electro-optic module 308 opposite outer surface 131 of window 13 on which sensing layer 36 (not shown) would be located. Optical body 388 has a generally truncated pyramid shape with the larger upper portion mating with housing 12 and surface 131 for supporting sensing layer 36, and opposed flat sides 348, 348'. Having the photodetectors 428, 448 arranged in opposing pairs increases the optical coupling through optical body 388 and provides a strong signal. As used herein, the term "truncated cone" is intended to include any of the shapes of body portions 385-388 of modules 305-308 shown in FIGS. 5-8 irrespective as to whether the sides of the truncated cone are curved, flat or a combination thereof.

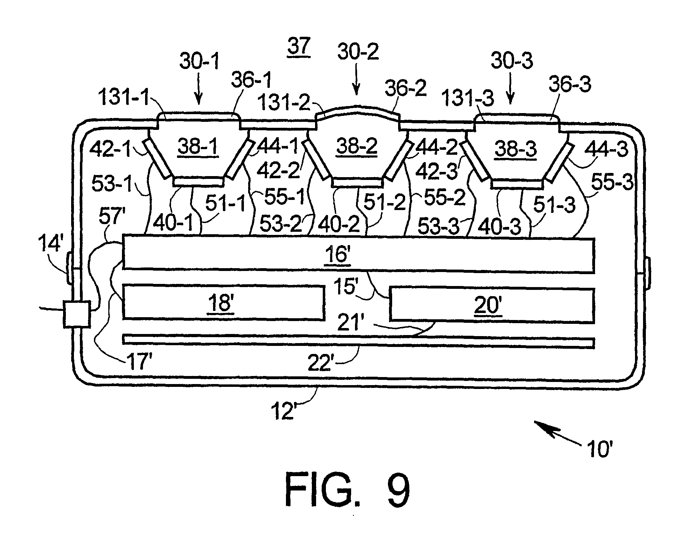

FIG. 9 is a simplified schematic cross-sectional view similar to FIG. 1, of implantable chemical sensor 10' according to a still further embodiment of the present invention. Chemical sensor 10' comprises hermetic enclosure or housing 12' that is sealable along joint 14'. Conveniently located within housing 12' are optical modules 30-1, 30-2, 30-3, electronic control module 16', energy source 18' (e.g., one or more batteries), optional transceiver 20' and optional antenna 22'. Optical modules 30-1, 30-2, 30-3 have therein light emitter(s) 40-1, 40-2, 40-3 coupled to control module 16' by leads 51-1, 51-2, 51-3, light detectors 42-1, 42-2, 42-3 and 44-1, 44-2, 44-3 coupled to control module 16' by leads 53-1, 53-2, 53-3 and 55-1, 55-2, 55-3, respectively, and other components as will be presently described. Control module 16' is coupled to energy source 18' by lead(s) 17' and to transceiver 20' by lead(s) 15'. Transceiver 20' is coupled to antenna 22' by lead(s) 21'. While transceiver 20' and antenna 22' are conveniently provided for communicating to and from sensor 10' they are not essential and externally coupled lead(s) 57' may also be used. Further, while it is desirable that instructions or other commands be able to be sent to sensor 10' while in situ using lead(s) 57' or antenna 22', this is not essential. Sensor 10' can be preprogrammed to merely report measured data after implantation without further instructions from outside.

Sensor 10' differs from sensor 10 of FIG. 1 in that sensor 10' has three optical modules 30-1, 30-2, and 30-3 therein, analogous to module 30 of FIG. 1 and modules 302-308 of FIGS. 2-8. While three optical modules are shown in FIG. 9, persons of skill in the art will understand that this is merely for convenience of explanation and that any number greater than one may be provided in sensor 10', consistent with the space limitations where sensor 10' is intended to be placed and the number of different analytes desired to be simultaneously detected or measured.

Modules 30-1, 30-2, and 30-3 are hermetically sealed to enclosure 12' and electrically coupled to control module 16'. Optical modules 30-1, 30-2, 30-3 have, respectively, optical bodies 38-1, 38-2, 38-3 analogous to optical body 38 of sensor 10, and corresponding light sources 40-1, 40-2, 40-3 and detectors 42-1, 42-2, 42-3 and 44-1, 44-2, 44-3 analogous to light source 40 and detectors 42, 44 of sensor 10 of FIG. 1. Connecting leads 51-1, 51-2, 51-3; 53-1, 53-2, 53-3; and 55-1, 55-2, 55-3 are analogous to leads 51, 53, and 55 of sensor 10 of FIG. 1. For simplicity, separate window(s) 13 and filters 50, 52, 54 are not shown in sensor elements 30-1, 30-2 and 30-3 of FIG. 9 but persons of skill in the art will understand that they can be included as desired. Temperature sensor [T], pressure sensor 56 and light beams 41, 43, 45 shown in FIG. 1 are omitted from FIG. 9 for clarity but this is not intended to be limiting or imply that such features or elements are not included.

Optical modules 30-1, 30-2, 30-3 have, respectively, outer surfaces 131-1, 131-2, 131-3 analogous to outer surface 131 of FIGS. 1-4, on which are located sensor layers 36-1, 36-2, and 36-3 equivalent to layers 36, 361-364 shown in FIGS. 1-4. Similarly, indicator molecules and reference molecules 60, 62 are omitted from FIG. 9 for simplicity and not intended to be limiting. Optical modules 30-1, 30-2, 30-3 perform substantially the same function as optical modules 30, 302-308 of FIGS. 1-8 in substantially the same way and the discussion and variations associated with FIGS. 1-8 are applicable to modules 30-1, 30-2, 30-3 and sensor 10' of FIG. 9. In FIG. 9, there is further illustrated in module 30-2, the optional use of non-planar outer surface 131-2 supporting sensor layer 36-2.

Multiple optical modules 30-1, 30-2, 30-3 in sensor 10' are conveniently used with sensor layers 36-1, 36-2, 36-3 of different composition, with different sensing and/or reference molecules. The wavelengths emitted by light sources 40-1, 40-2, 40-3 and the filters used with light sources 40-1, 40-2, 40-3 and with detectors 42-1, 42-2, 42-3 and 44-1, 44-2, 44-3 can be appropriately modified for the different sensing layers intended to respond to different analytes. In this way, different analytes of ambient 37 may be separately monitored at the same time using single implantable sensor 10'. Further, sensor molecules and reference molecules may be place on different sensor elements rather than combined on the same sensor element in situations where they might undesirably interact. Thus, the use of multiple optical modules in the same housing can greatly enhance the ability to monitor important biological and/or chemical activity in situ in a human or animal body. As used herein, the word "animal" is intended to include any non-human animate organism on land or in the sea or air. Persons of skill in the art will also understand that while the present invention is particularly useful in connection with living organisms, it may also be used in any situation where in situ measurement or detection of particular analytes is desired and relevant sensor molecules are available.

## Claims

1. A chemical sensor for detecting or measuring a parameter of interest in a sample fluid, comprising:
a housing (12) chemically resistant to the sample fluid;
a sensor element (30) substantially within the housing, the sensor element having a sensing layer (36) and an optically transparent body (38) with a first surface exposed from the housing for receiving a sample responsive to the parameter of interest in the sample fluid and for receiving a first signal from the sensing layer in response to optical stimulation thereof;
wherein the sensor element comprises a light emitter (40) on a second surface of the body, optically coupled to the first surface for providing said optical stimulation;
wherein the sensor element comprises a first detector (42, 44) on a third surface of the body, optically coupled to the first surface for detecting said first signal; and **characterized in that** of the first, second and third surfaces of the body, two surfaces are opposed and a remaining surface couples the opposed surfaces.

2. The sensor of claim 1 wherein the first surface is adapted to receive a further response from a reference component (62) associated with the sensing layer (36) for providing, in response to optical stimulation, a second signal unaffected by the parameter of interest.

3. The sensor of claim 2 further comprising a second detector (44, 42) on a fourth surface of the body (38), optically coupled to the first surface for responding to the second signal.

4. The sensor of any of the claims 1 to 3 further comprising a control module (16) located within the housing (12) and coupled to the sensor element (30) for actuating said stimulation and measuring said first signal.

5. The sensor of any of the claims 1 to 4 further comprising a temperature or pressure sensor coupled to the control module.

6. The sensor of any of the claims 1 to 5 further comprising:
a control module (16) located within the housing (12) and coupled to the sensor element for actuating said stimulation and measuring said first signal;
a transmitter (20) located within the housing and coupled to the control module; and
an antenna (22) located within the housing and coupled t o the transmitter for coupling the results of measuring the first signal outside the module.

7. The sensor of any of the claims 1 to 6 further comprising a second sensor element substantially within the same housing (12), the second sensor element having an optically transparent second body with a first surface exposed from the housing for receiving a second layer responsive to a different parameter of interest in the sample fluid and for receiving a first signal from the second sensing layer in response to optical stimulation thereof; wherein the sensor element comprises a light emitter on a second surface of the second body, optically coupled to the first surface for providing said optical stimulation; wherein the second sensor element comprises a first detector on a third surface of the second body, optically coupled to the first surface for detecting said first signal; and wherein of the first, second and third surfaces of the second body, two surfaces are opposed and a remaining surface couples the opposed surfaces.

## Patentansprüche

1. Chemischer Sensor zum Erfassen oder Messen eines interessierenden Parameters in einem Probenfluid, mit:
einem Gehäuse (12), das gegenüber dem Probenfluid chemisch resistent ist;
einem Sensorelement (30), das sich im Wesentlichen in dem Gehäuse befindet, wobei das Sensorelement eine Erfassungsschicht (36) und einen optisch transparenten Körper (38) mit einer von dem Gehäuse freigegebenen bzw. exponierten Oberfläche zum Aufnehmen einer auf den interessierenden Parameter ansprechenden Probe in dem Probenfluid und zum Empfangen eines ersten Signals von der Erfassungsschicht in Reaktion auf deren optische Stimulation besitzt;
wobei das Sensorelement einen Lichtsender bzw. -emitter (40) auf einer zweiten Oberfläche des Körpers, die mit der ersten Oberfläche optisch gekoppelt ist, aufweist, um die optische Stimulation bereitzustellen;
wobei das Sensorelement einen ersten Detektor (42, 44) auf einer dritten Oberfläche des Körpers, die mit der ersten Oberfläche optisch gekoppelt ist, aufweist, um das erste Signal zu erfassen; und **dadurch gekennzeichnet, dass** von der ersten, der zweiten und der dritten Oberfläche des Körpers zwei Oberflächen einander gegenüberliegen und eine verbleibende Oberfläche die gegenüberliegenden Oberflächen koppelt.

2. Sensor nach Anspruch 1, wobei die erste Oberfläche so beschaffen ist, dass sie eine weitere Antwort von einer Referenzkomponente (62) empfängt, die der Erfassungsschicht (36) zugeordnet ist, um in Reaktion auf eine optische Stimulation ein zweites Signal bereitzustellen, das durch den interessierenden Parameter nicht beeinflusst ist.

3. Sensor nach Anspruch 2, der ferner einen zweiten Detektor (44, 42) auf einer vierten Oberfläche des Körpers (38), die mit der ersten Oberfläche optisch gekoppelt ist, aufweist, um auf das zweite Signal anzusprechen.

4. Sensor nach einem der Ansprüche 1 bis 3, das ferner ein Steuermodul (16) aufweist, das sich in dem Gehäuse (12) befindet und mit dem Sensorelement (30) gekoppelt ist, um die Stimulation hervorzurufen und um das erste Signal zu messen.

5. Sensor nach einem der Ansprüche 1 bis 4, das ferner einen Temperatur- oder Drucksensor, der mit dem Steuermodul gekoppelt ist, aufweist.

6. Sensor nach einem der Ansprüche 1 bis 5, ferner mit:
einem Steuermodul (16), das sich in dem Gehäuse (12) befindet und mit dem Sensorelement gekoppelt ist, um die Stimulation hervorzurufen und das erste Signal zu messen;
einem Sender (20), der sich in dem Gehäuse befindet und mit dem Steuermodul gekoppelt ist; und
einer Antenne (22), die sich in dem Gehäuse befindet und mit dem Sender gekoppelt ist, um die Ergebnisse der Messung des ersten Signals mit der äußeren Umgebung des Moduls zu koppeln.

7. Sensor nach einem der Ansprüche 1 bis 6, ferner mit einem zweiten Sensorelement, das sich im Wesentlichen im selben Gehäuse (12) befindet, wobei das zweite Sensorelement einen optisch transparenten zweiten Körper mit einer von dem Gehäuse freigegebenen ersten Oberfläche zum Empfangen einer auf einen anderen interessierenden Parameter in dem Probenfluid ansprechenden zweiten Probe und zum Empfangen eines ersten Signals von der zweiten Erfassungsschicht in Reaktion auf deren optische Stimulation besitzt; wobei das Sensorelement einen Lichtsender auf einer zweiten Oberfläche des zweiten Körpers, die mit der ersten Oberfläche optisch gekoppelt ist, um die optische Stimulation zu schaffen, aufweist; wobei das zweite Sensorelement einen ersten Detektor auf einer dritten Oberfläche des zweiten Körpers, die mit der ersten Oberfläche optisch gekoppelt ist, um das erste Signal zu erfassen, aufweist; und wobei von der ersten, der zweiten und der dritten Oberfläche des zweiten Körpers zwei Oberflächen einander gegenüberliegen und eine verbleibende Oberfläche die gegenüberliegenden Oberflächen koppelt.

## Revendications

1. Capteur chimique pour détecter ou mesurer un paramètre d'intérêt dans un fluide échantillon, comportant :
une enceinte (12) résistant chimiquement au fluide échantillon ;
un élément de capteur (30) se trouvant essentiellement dans l'enceinte, l'élément de capteur ayant une couche de détection (36) et un corps optiquement transparent (38) avec une première surface exposée depuis l'enceinte pour recevoir un échantillon sensible au paramètre d'intérêt dans le fluide échantillon et pour recevoir un premier signal depuis la couche de détection en réponse à une stimulation optique de celle-ci ;
dans lequel l'élément de capteur comporte un émetteur de lumière (40) sur une deuxième surface du corps, couplée optiquement à la première surface pour fournir ladite stimulation optique ;
dans lequel l'élément de capteur comporte un premier détecteur (42, 44) sur une troisième surface du corps, couplée optiquement à la première surface pour détecter ledit premier signal ; et **caractérisé en ce que** parmi les première, deuxième et troisième surfaces du corps, deux surfaces sont en vis-à-vis et une surface restante couple les surfaces en vis-à-vis.

2. Capteur selon la revendication 1, dans lequel la première surface est adaptée pour recevoir une réponse supplémentaire depuis un composant de référence (62) associé à la couche de détection (36) pour fournir, en réponse à une stimulation optique, un second signal non affecté par le paramètre d'intérêt.

3. Capteur selon la revendication 2, comportant de plus un second détecteur (44, 42) sur une quatrième surface du corps (38), couplée optiquement à la première surface pour répondre au second signal.

4. Capteur selon l'une quelconque des revendications 1 à 3, comportant de plus un module de commande (16) positionné à l'intérieur de l'enceinte (12) et couplé à l'élément de capteur (30) pour actionner ladite stimulation et mesurer ledit premier signal.

5. Capteur selon l'une quelconque des revendications 1 à 4, comportant de plus un capteur de température ou de pression couplé au module de commande.

6. Capteur selon l'une quelconque des revendications 1 à 5, comportant de plus :
un module de commande (16) positionné à l'intérieur de l'enceinte (12) et couplé à l'élément de capteur pour actionner ladite stimulation et mesurer ledit premier signal ;
un émetteur (20) situé à l'intérieur de l'enceinte et couplé au module de commande ; et
une antenne (22) située dans l'enceinte et couplée à l'émetteur pour coupler les résultats de mesure du premier signal à l'extérieur du module.

7. Capteur selon l'une quelconque des revendications 1 à 6, comportant de plus un second élément de capteur se trouvant essentiellement dans la même enceinte (12), le second élément de capteur ayant un second corps optiquement transparent ayant une première surface exposée depuis l'enceinte pour recevoir une seconde couche sensible à un paramètre d'intérêt différent dans le fluide échantillon et pour recevoir un premier signal depuis la seconde couche de détection en réponse à une stimulation optique de celle-ci ; dans lequel l'élément de capteur comporte un émetteur de lumière sur une deuxième surface du second corps, couplée optiquement à la première surface pour fournir ladite stimulation optique ; dans lequel le second élément de capteur comporte un premier détecteur sur une troisième surface du second corps, couplée optiquement à la première surface pour détecter ledit premier signal ; et dans lequel parmi les première, deuxième et troisième surfaces du second corps, deux surfaces sont en vis-à-vis et une surface restante couple les surfaces en vis-à-vis.
